(19)

**Europäisches Patentamt**
**European Patent Office**
**Office européen des brevets**

(11) **EP 4 736 845 A1**

(12) **EUROPEAN PATENT APPLICATION**

(43) Date of publication:
06.05.2026 Bulletin 2026/19

(21) Application number: 24306864.0

(22) Date of filing: 05.11.2024

(51) International Patent Classification (IPC):
*A61K 9/16* (2006.01)  *A23L 33/135* (2016.01)

(52) Cooperative Patent Classification (CPC):
A61K 9/1682; A23L 33/135; A23L 33/21;
A61K 9/1652

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(71) Applicants:
• **Institut National de la Recherche pour l'Agriculture, l'Alimentation et l'Environnement**
75007 Paris (FR)
• **Institut National des Sciences et Industries du Vivant et de l'Environnement**
91120 Palaiseau (FR)
• **Université Paris-Saclay**
91190 Gif-sur-Yvette (FR)
• **Instituto Politecnico Nacional**
Zacatenco Ciudad De México, 07738 (MX)

(72) Inventors:
• **GARFIAS-NOGUEZ, Cynthia**
54680 State of Mexico (MX)
• **RAMÍREZ-DAMIÁN, Morayma**
13020 Mexico City (MX)
• **SÁNCHEZ-PARDO, María Elena**
06400 Mexico City (MX)
• **BERMÚDEZ-HUMARÁN, Luis**
91400 SACLAY (FR)
• **ALAMILLA-BELTRÁN, Liliana**
06720 Mexico City (MX)

(74) Representative: **Santarelli**
Tour Trinity
1 bis Place de la Défense
92400 Courbevoie (FR)

(54) **SPRAY DRIED SYNBIOTIC MICROCAPSULES OF ENHANCED STORAGE STABILITY AND PROBIOTIC ACTIVITY**

(57) The present invention concerns novel synbiotic microcapsules of enhanced storage stability at room temperature. The symbiotic microcapsules are produced in a single step by spray drying, and present a microcapsule composition consisting essentially of a probiotic, and a mixture of a prebiotic preferably inulin, maltodextrin, and a protective colloid selected from the group consisting of gum Arabic, starch, rice starch, modified starches, pectin, xanthan gum and mixtures thereof. The invention also proposes a spray drying process easy to operate and providing excellent encapsulation and efficiency yields. The synbiotic microcapsules have proven to effectively protect the encapsulated probiotic during gastrointestinal transit and to provide a probiotic effect on *in vitro* and *in vivo* essays.

**EP 4 736 845 A1**

**Description**

**TECHNICAL FIELD**

The present invention concerns new spray dried synbiotic microcapsules and a process for microencapsulation thereof. In particular, the invention proposes new microcapsules comprising a mixture of probiotics and prebiotics (i.e. synbiotic) exhibiting enhanced storage stability without the need for refrigeration. The invention also proposes a spray drying process providing excellent encapsulation and efficiency yields. The synbiotic microcapsules have proven to effectively protect the encapsulated probiotic during gastrointestinal transit and to provide a probiotic effect on *in-vitro* and *in-vivo* essays.

**STATE OF THE ART**

Probiotics are live microorganisms that, when administered in adequate amounts, offer health benefits to the host (FAO/WHO, 2002). Among these, lactic acid bacteria (LAB) such as lactobacilli, streptococci, and enterococci, along with bifidobacteria, are the most extensively studied genera of probiotics. These microbes not only enhance sensory qualities and produce organic acids and bacteriocins during food fermentation, but also significantly benefit intestinal functions. They contribute to balancing intestinal microbiota, regulating intestinal transit, maintaining acid-base balance in the colon, and modulating immune responses. Additionally, they exhibit antimicrobial, antioxidant, and anti-inflammatory effects. For example, probiotics are being studied as alternative treatment to chronic inflammatory bowel disease (IB) like ulcerative colitis, which presents a complex multifactorial etiopathogenesis and wherein progression is primarily characterized by dysregulated immune responses and dysfunctions in the intestinal barrier which some probiotic can help to restore.

The beneficial impacts of probiotics often stem from their ability to survive in the gastrointestinal tract, adhere to epithelial surfaces, and modulate immune systems. To ensure the efficacy of probiotics, strategies aiming to enhance the viability of these bacteria are being explored. Among them, encapsulation has emerged as one of the main techniques. This process involves encasing the probiotic microorganism in a protective coating matrix that remains stable and resistant until it releases its content in specific environments. This encapsulating strategy enhances protection of probiotics during manufacturing, storage, and passage through the gastrointestinal tract.

The international patent application WO 2019/144979 discloses a method of coating and encapsulation for probiotics which allows for extended and stable storage of such products without the need for refrigeration. A two-step method is proposed wherein a probiotic core is first carried out using a fluid bed and a binder solution (gum Arabic) with stabilizing materials (trehalose, vitamin E acetate, and whole milk powder). In a second step, the obtained granules are coated by spraying a film-forming solution consisting of polyvinyl alcohol in water. With a total process time of around 300 minutes, this process and formulation allows obtaining probiotic microcapsules with a viability of up to $10^9$ CFU/g after 12 months. However, the processing time is long and the formulation requires several ingredients, including stabilizers and a coating polymer.

Spray drying is an alternative method for microencapsulating probiotics in a single step. Spray drying involves atomizing a probiotic suspension or solution-comprising both bacteria and the encapsulating material-into fine droplets within a hot air stream. This causes rapid solvent evaporation, resulting in the formation of microencapsulated solid particles. However, during spray-drying process, the membrane of probiotic cells gets damaged, and as a result, they are vulnerable to heat and dehydration. Careful set-up of the process parameters and proper formulation is therefore needed to protect the probiotics during air drying.

The international patent application WO 2015/063090 gives an example of a spray drying microencapsulation method for lactic acid bacteria using GEA Niro Mobile Minor spray drying. An inlet temperature of 66°C and an outlet temperature of 39°C were used, with nitrogen as the heating fluid. Maltodextrin 12 DE, inulin, trehalose dihydrate, and sodium ascorbate were utilized, with a total solid's percentage of 51%. In particular, it is suggested to keep the sprayed particles for maximum 20 minutes in the spray dryer, and to convey the sprayed dried material to a secondary cyclone separator by means of a cooling gas. They achieved a bacterial concentration of 2.0E+11 CFU/g $\pm$15% with drying yields of 90%, moisture content of 7.5% and water activity of about 0.24. After three weeks, viability decreased to 8E+10 CFU/g. Although the formulation and process allow a high encapsulation efficiency, the microcapsules lose an order of magnitude in just three weeks. Moreover, the method uses nitrogen as heating fluid (elevating production costs) and the formulation requires stabilizers.

Arepally et al., 2020 [1] conducted studies on viability and storage stability of encapsulated dried probiotic powder. In this study, the probiotic microorganism (*i.e. Lactobacillus acidophilus)* were encapsulated by spray drying technology to produce a probiotic powder using 20% of maltodextrin and varied concentrations of gum Arabic. The effect of processing conditions such as inlet air temperature (130-150°C) and gum Arabic concentration (0-10%) on the encapsulation efficiency and physical properties were studied. They mention that an increase in inlet temperature decreases encapsulation yield, probably because of thermal and dehydration inactivation of cells. Increasing the concentration of gum Arabic allowed to increase encapsulation efficiency, which is considered the most important factor in the microencapsulation of probiotics as it reflects the bacterial viability. The survivability of encapsulated *L acidophilus* during storage was also determined. Microcapsules were obtained with an inlet temperature of 150°C and 8.51 % of gum Arabic, and with an outlet air temperature recorded at 55°C. The microencapsulated probiotics have an initial viability count of around 9 log CFU/g which decreases to around 5 log CFU/g after 12 weeks when stored at 25°C. Such conditions provide a high encapsulation efficiency with a simple formulation, however, the storage stability at room temperature is poor.

Avila-Reyes et al., 2014[2] suggested to use prebiotics in order to enhance the viability of spray dried probiotics during storage. Prebiotics are defined as non-digestible food ingredients that promote the growth of probiotics. The combination of probiotics and prebiotics in a same product is called synbiotic. It is assumed that the viability of the probiotic will be increased when it reaches the colon, as it could use the prebiotic for survival. Avila-Reyes et al. 2014 studied the protective effect of inulin and corn starch as wall materials (10-20%) on viability of [2] *Lacticaseibacillus rhamnosus* microencapsulated by spray-drying. They found that inulin and rice starch can be used as a single wall material ingredient for protecting probiotics encapsulated by spray drying when using an inlet temperature of 135-155 ° C, and recorded outlet temperatures of 58-77 °C. Inulin provides however lower encapsulation yields and also provides inferior protective effects when compared to rice starch. The viability of the microcapsules was reduced of 2,26 log after 5 weeks of storage at 25°C.

New probiotic microcapsules of optimal stability under storage at room temperature are therefore still needed. Such microcapsules shall also provide effective protection of the probiotics during manufacturing and in gastrointestinal conditions, as well as maintain a probiotic effect when reaching the colon. It will also be optimal that such microcapsules can be obtained by a simple process and formulation. The present application aims to provide such microcapsules and process.

## SUMMARY OF THE INVENTION

The present invention concerns novel synbiotic microcapsules of enhanced storage stability at room temperature. The symbiotic microcapsules are produced in a single step by spray drying, and present a microcapsule composition which is simple, low cost and does not require stabilizers or coating films to ensure storage stability.

The present invention is defined in the appended set of claims.

In particular, the microcapsule composition consists essentially of a probiotic, and a mixture of:

- a prebiotic selected from the group consisting of fructooligosaccharides (FOS), galactooligosaccharides (GOS), xylooligosaccharides (XOS), and arabinoxylans and mixtures thereof;

- maltodextrin, and

- a protective colloid selected from the group consisting of gum Arabic, starch, rice starch, modified starches, pectin, xanthan gum and mixtures thereof.

Specifically, the prebiotic considerably increases the storage stability of the microcapsules when mixed with maltodextrin and a protective colloid of carbohydrate polymer nature. Such effect appears to be related to water behavior in the composition, and wherein such mixture of components is able to provide microcapsules of relatively constant moisture content (4-6%) and low water activity (<0.4, notably <0.35), even when used in different compositions varying in ingredient concentrations, solid content and temperature of processing. In a preferred embodiment, the hygroscopic probiotic is a FOS, preferably inulin.

The microcapsule composition does not require additional stabilizers or additives in order to exhibit optimal storage stability at room temperature. The microcapsule composition is therefore said to "consist essentially of a probiotic, a prebiotic, maltodextrin and a protective colloid". However, a synbiotic microcapsule composition including additional ingredients which do not modify the storage stability are to be considered within the scope of the claim. Microcapsules compositions with additional ingredients known to promote stability, but wherein such ingredients are present bellow their required concentration to promote stability shall also be considered within the scope of the claim.

In an embodiment, the mixture of the prebiotic, maltodextrin and the protective colloid represent at least 90% w/v of the total solids (excluding the probiotic) of the composition when present in aqueous solution/dispersion, preferably at least 95% w/v of the total solids, more preferably at least 96%, 97%,98% or 99% w/v of the total solids.

For example, the microcapsules have the following composition in w/v % when present in aqueous solution:

1 - 10% of prebiotic preferably excluding limit values, more preferably 2-8%, 3-7 %, or 4-6%;
5 - 15% of maltodextrin, preferably 5-10%
5 - 15% of protective colloid, preferably 5-10%; and

wherein the total solid content in such aqueous solution, excluding the probiotic, is 20 - 30 %w/v, preferably 20-25% w/v.

In a preferred embodiment, the preferred range of concentration values of the composition are selected so as to provide optimal encapsulation efficiency, and encapsulation yield.

The invention also relates to food compositions, nutraceutical compositions, dietary supplement compositions or medicament compositions including the synbiotic microcapsules of the invention, and to their use in the treatment and/or prevention of chronic inflammatory bowel disease, such as ulcerative colitis. A food composition is defined as any composition for ingestion by humans or animals, comprising one or more edible substances which may include macronutrients (such as proteins, carbohydrates, and fats), micronutrients (such as vitamins and minerals), and that may further include additives, preservatives, or other ingredients to ensure stability, shelf-life, and consumer acceptability. For example, the food composition may be selected from a diary product, a beverage, snack bars, flour, any bakery product, infant formula, candies, capsules, tablets, powders, and any oral formulation. The food product may also be presented in various physical forms, including but not limited to liquids, solids, semi-solids, powders, or gels, and is designed to provide nutritional, functional, or sensory benefits when consumed.

A food composition including the synbiotic microcapsules of the invention may be also referred as "functional food" or "nutraceutical" as the composition will provide health benefits beyond basic nutritional value. The synbiotic microcapsules may also be added in other functional food or nutraceutical compositions by combining the microcapsules of the invention in other compositions including one or more bioactive ingredients that positively influence physiological functions or reduce the risk of disease, such as, but not limited to vitamins, minerals, antioxidants, prebiotics, probiotics, fibers, polyphenols, peptides, or other nutraceutical compounds.

By addition of the synbiotic microcapsules, such compositions will provide health benefits such as balancing intestinal microbiota, regulating intestinal transit, maintaining acid-base balance in the colon, modulating immune responses, acting as antioxidants, antimicrobials and antiinflammatories, as well as treating and preventing gastrointestinal diseases and disorders such colorectal cancer, and chronic inflammatory bowel disease (IB) like ulcerative colitis.

The synbiotic microcapsules may also be included in medicament or pharmaceutical compositions by association with a pharmaceutically suitable vehicle. A "pharmaceutically suitable vehicle" herein means a vehicle that is useful in preparing a pharmaceutical composition or formulation and that is generally safe, non-toxic, and neither biologically nor otherwise undesirable for the subject to be treated, in particular mammals and more particularly humans. The vehicle of the composition of the invention may be solid, semi-solid or liquid.

It may be a diluent, an adjuvant or any other vehicle conventional per se for the constitution of pharmaceutical compositions. The pharmaceutical composition of the invention may be formulated in any galenical form, in particular in a form that is suitable for administration in mammals, and in particular in humans. It is preferably formulated in a form which is suitable for administration by oral route, such as a form powder, capsules, oral solution or suspension, or in a form which is suitable for administration by topical route, especially on the skin and/or the mucous of the subject, such as a cream, etc. The pharmaceutical composition of the invention may comprise one or more excipients / additives conventional by themselves, for example preservatives, sweetening agents, flavoring agents, suspending agents, dispersing agents, lubricating agents, stabilizing agents, buffering agents, or any of their mixtures. It may also contain one or more other active agents, which may or may not act in synergy with the microcapsule of the invention.

The invention also concerns a process for obtaining the synbiotic microcapsules by spray drying. In a preferred embodiment, the inlet temperature is set at a temperature of about 130°C to 170°C, preferably 145°C to 165°C, and an outlet temperature of about 65°C to 85°C, preferably of about 70°C to 80°C. The process allows therefore to obtain stable synbiotic microcapsules in a wide range of temperatures, and is therefore simple to operate. The process can be operated in a semi-industrial equipment at rates of 10 to 27 mUmin, production rates are therefore convenient.

Further features and advantages of the invention will be apparent from the following description, which refers to examples of a non-limiting embodiment of the objects of the invention.

## FIGURES

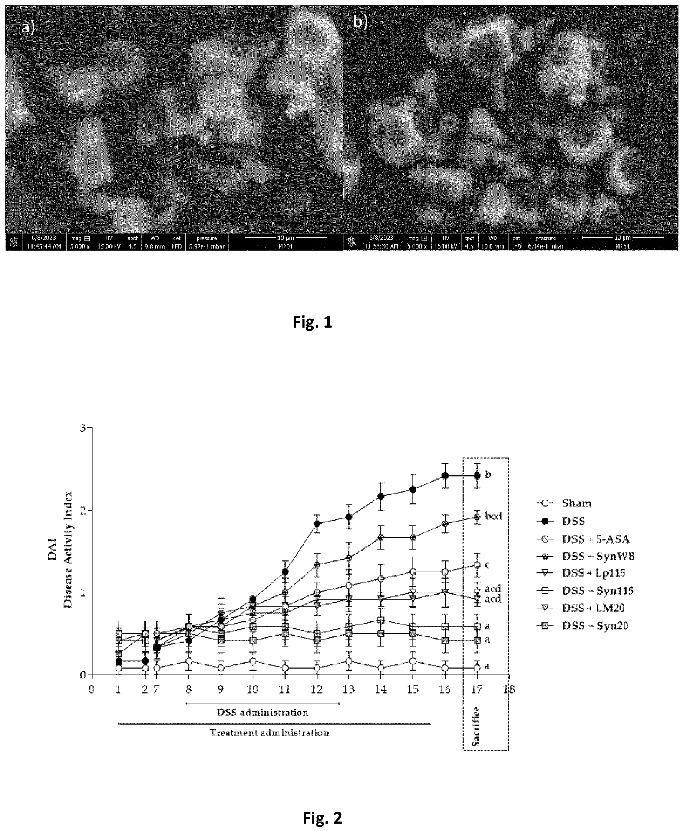

Figure 1: Microscopic morphology of (a) LM20 and (b) Lp115 symbiotic microcapsules according to example 1 observed in Scanning Electron Microscopy (FEI-Thermo-Fisher, ESEM Quanta FEG 250, US), under a voltage of 15 kV (kilovolts) and an increase of 5000X., The particle size was determined using the imaged program (version 1.53t) (National Institutes of Health, USA).

Figure 2: The evaluation of the Disease Activity Index (DAI) for ulcerative colitis was conducted in a murine model. The disease was induced by administering sodium dextran sulfate (DSS) of colitis grade. DAI is an average value obtained from the sum of weight loss, stool consistency, and the presence of bleeding in the experimental animals during the testing period. "Sham" refers to the healthy control group; "DSS" refers to the sick group (all groups with these initials indicate that they received doses of DSS); "DSS+5-ASA" is the control group that receives mesalamine as treatment; "DSS+SynWB" is the control group that receives microcapsules without bacteria; "DSS+LM20" and "DSS+LP115" are control groups that receive non-microencapsulated bacteria; "DSS+SynLM20" and "DSS+SynLp115" are control groups that receive microencapsulated bacteria (synbiotic), according to example 1 of the present invention. From day 1 to day 7 (the day before administration of DSS) no significant differences were observed, nor during the first 3 days after administration of DSS. From day 12 onwards, significant differences were observed in the study groups, but for interesting and visual purposes, the results shown correspond to those obtained on the day of sacrifice, n = 12. The data is represented as a mean $\pm$ standard error. Letters indicate significant difference: bars labeled with the same letters are not significantly different from each other, while those labeled with different letters indicate a significant difference $p \leq 0.05$ from each other

## DETAILED DESCRIPTION

The aim of the present invention is to propose novel synbiotic microcapsules produced by spray-drying. These microcapsules are classified as synbiotic due to their integration of both a probiotic and a prebiotic, notably lactic acid bacteria and/or bifidobacteria. The invention also concerns a spray drying method for producing the synbiotic microcapsules, and the use of the synbiotic microcapsules in food products and medicaments, as well as for the treatment and prevention of inflammatory bowel diseases, such as ulcerative colitis.

Specifically, the present invention proposes a novel microcapsule composition offering significant advantages, as it ensures excellent storage stability at room temperature with a simple and low-cost formulation. This makes the microcapsules easy to manufacture, manage, and store, enhancing their practical utility.

In a preferred embodiment, the synbiotic microcapsules composition consists essentially of:

- a probiotic, such as lactic acid bacteria and/or bifidobacteria;
- a prebiotic selected from the group consisting of fructooligosaccharides (FOS), galactooligosaccharides (GOS), xylooligosaccharides (XOS), and arabinoxylans and mixtures thereof;
- maltodextrin, and
- a protective colloid selected from the group consisting of gum Arabic, starch, rice starch, modified starches, pectin, xanthan gum and mixtures thereof.

Maltodextrin is widely used as a wall material in microencapsulation because it has excellent film-forming properties, has good compatibility with other materials and is wide available. Mixing maltodextrin with a protective colloid is known to increase thermal protection during spray drying, increasing encapsulation efficiency and stability during storage of different food products.

In the present invention, the inventors found that adding a hygroscopic polymeric prebiotic (such as defined before) to said mixture of wall materials, dramatically increases the storage stability of the microcapsules at room temperature.

Indeed, the inventors tested the storage stability of different probiotic strains microencapsulated according to the invention, notably *Lacticaseibacillus rhamnosus, Levilactobacillus brevis, Lactiplantibacillus plantarum* (Experimental part, Table 4). For all tested strains, the viability of the synbiotic microcapsules decreases no more than 3 logs after 12 months of storage in a desiccator at 25°C.

Without being bound by the present theory, it is believed that such enhanced stability is related to water behavior in the composition, wherein the addition of the prebiotic regulates the moisture content and water activity of the microcapsules (Experimental part, tables 1 and 2). As it will be further explained in the experimental part, the invention provides microcapsules with a desired combination of relatively high moisture content (4.12-5.63%) and low water activity (0.21-0.32) which appears to provide enhance storage stability.

The microcapsules are obtained by spray drying with high encapsulation efficiencies and encapsulation yields, so that synbiotic microcapsules with a high initial viability can be manufactured, and wherein the viability does not decrease more than 4 logs after 12 months of storage in desiccator at 25°C, preferably no more than 3 logs.

Specifically, the synbiotic microcapsules are produced by air spray-drying with an inlet temperature of about 130°C to 170°C, preferably 145°C to 165°C, and an outlet temperature of 65°C to 85°C, preferably 70°C to 80°C. Such processing conditions lead to excellent encapsulation efficiency and encapsulation yields (see experimental part table 1). The inlet temperature of about 160°C $\pm$ 5 °C is preferred for elevating the encapsulation efficiency and yield (Table 2).

The symbiotic microcapsules of the invention are said to be microcapsules without reference to a particular size or microcapsule structure. This is, any particle obtained by spray drying the microcapsule composition of the invention is considered to be a microcapsule, irrespectively of the structure of such microcapsule.

Fig. 1 shows an image of synbiotic microcapsules of *Lactiplantibacillus plantarum* LM20 (a) and Lp115 (b).

Wherein the microcapsules display irregular shape (almost spherical) with variable sizes ranging from 2.8 to 7.4 $\mu$m. It is not known if the wall materials form a coating surrounding the bacteria or if some bacteria are present at the exterior surface of the microcapsules.

According to one embodiment, the microcapsules have the following composition in w/v % when present in aqueous solution:

1 - 10% of prebiotic preferably excluding limit values, more preferably 2-8%, 3-7 %, or 4-6%;
5 - 15% of maltodextrin, preferably 5-10%
5 - 15% of protective colloid, preferably 5-10%; and

wherein the total solid content in such aqueous solution, excluding the probiotic, is 20 - 30 %w/v, preferably 20-25% w/v.

Unless otherwise specified, the range of concentrations include limit values. In particular, when the concentration of the prebiotic is at least 1% w/v, the water activity of the synbiotic microcapsules is low, and the microcapsules will have increased storage stability.

Preferably, in order to obtain optimal values of encapsulation efficiency and efficiency yield, as well as to ensure smooth processing, the concentration of prebiotic in the aqueous dispersion before drying shall be > 1 % w/v and <10%w/v, wherein results are optimized in the cited above preferred range of values. The preferred concentration ranges of maltodextrin and protective colloid also enhance encapsulation efficiency and efficiency yield, however they are not essential to enhance stability.

According to an embodiment, the lower ranges of some of the components are selected as to reduce formulation costs. The composition will also be balanced so as to obtain a liquid dispersion of a viscosity adapted to be spray dried, and to match the total required solid content.

In a preferred embodiment the prebiotic is inulin, and the microcapsules have the following composition in w/v % when present in aqueous solution:

3 - 7 % if inulin as prebiotic, preferably 4-6 %;
5 - 15% of maltodextrin, preferably 5-10%;
5 - 10% of gum Arabic as protective colloid, and

wherein the total solid content in such aqueous solution, excluding the probiotic, is 20-25% w/v.

As it will be further detailed in the experimental part, results section, the inventors proved that the synbiotic microcapsules are able to protect the probiotic during gastrointestinal simulated transit conditions (Table 5).

Finally, the inventors also corroborated the probiotic effect of the synbiotic microcapsules on human adenocarcinoma cell lines. Specifically, all tested microencapsulated probiotics showed adhesion capacity, reflecting their ability to colonize HT-29 cells and their potential to be used as a treatment for targeting tumors. It was also outlined the capacity of the microcapsules to modulate tumor cell proliferation and IL-8 expression (Table 6).

An in-vivo experiment was also conducted in a murine model of ulcerative colitis (DSS) (see figure 2). In such experiment it was demonstrated that administering *Lactiplantibacillus plantarum* LM-20 microcapsules according to example 1 was able to decrease the disease activity index (DAI) to values close of such of the healthy control group (Sham), notably after 17 days of treatment. The synbiotic microcapsules of LM20 (Syn) also provided a superior effect when compared to the ingestion of the free bacteria (LM20).

The results highlight that the synbiotic microcapsules of the invention are able not only to protect and preserve the probiotic during manufacturing, storage, and gastrointestinal transit, but they also enhance the probiotic effect when administered as a therapeutic treatment.

EXPERIMENTAL PART

I. MATERIALS AND METHODS

1. Materials

The following probiotic bacterias were studied:

-    *Lactiplantibacillus plantarum* Lp-115 ATCC SD5209 (Danisco, Brabrand, Denmark), code in the present invention: Lp115
-    *Lactiplantibacillus plantarum* LM-20 CDBB-B-2104 (CINVESTAV, Zacatenco, Mexico City), code in the present invention: LM20, isolated by (Hernandez-Delgado et al., 2021) from the solid residue fermented from agave (*Agave angustifolia* Haw), obtained during the production of mezcal, in the locality of Macuilxóchilt de Ártiagas, in the State of Oaxaca, Mexico.

- *Lactiplantibacillus plantarum* LM19, isolated by (Hernandez-Delgado *et al.,* 2021[3]) from the solid residue fermented from agave (*Agave angustifolia* Haw), obtained during the production of mezcal, in the locality of Santiago Matatlán, in the State of Oaxaca, Mexico.
- *Lacticaseibacillus rhamnosus* LM07, isolated by (Hernandez-Delgado *et al.,* 2021[3]) from the solid residue fermented from agave (*Agave angustifolia* Haw), obtained during the production of mezcal, in the locality of Tlacolula de Matamoros, in the State of Oaxaca, Mexico.
- *Levilactobacillus brevis* LBH1070, isolated by (Torres-Maravilla *et al.,* 2016[4]) Sediments accumulated during the fermentation of pulque (xaxtle), in the locality of Milpa Alta, in the State of Mexico.

The prebiotic used was:

- Standard inulin, Orafti®GR (Azelis Mexico, SA de CV)

The coating material used was a combination of polysaccharides.

- Maltodextrin DE10 (Globe® Plus)
- Gum Arabic (Meyer® Lot: TG0914452)

The bacterial biomass of each probiotic strain was obtained from bacterial growth inoculated in Man, Rogosa, and Sharpe broth (MRS) and incubated for 48 hours at 37°C. Subsequently, it was centrifuged at $12,560 \times g$ at 4°C and resuspended in phosphate-buffered saline (PBS) solution.
The concentration of viable bacteria (bacterial viability) was determined by standard plate counting method and reported in CFU/g.

2. Synbiotic microcapsules

*Composition*

The composition prior to spray drying is an aqueous dispersion including a mixture of bacteria, prebiotics (2-10% w/v) and wall materials: maltodextrin 5-15% w/v and protective colloid 5-15% w/v. The wall materials and the prebiotic were dissolved in sterile purified water supported by a magnetic stirrer bar in a hotplate magnetic stirrer (Metrix®, model 58-H4000-HS, Mexico). Maltodextrin (MD) was hydrated for 24 h prior to drying process. Finally, 1 h before starting the drying process, a bacterial suspension was incorporated into the dispersion under constant stirring with the support of a magnetic stirrer bar in a hotplate magnetic stirrer. The final volume of the microencapsulating synbiotic solution was 300 mL, with a percentage of total solids of *20-30 % and a viable bacterial concentration of $10^{11}$ CFU/g of the solution.*

*Air drying process*

The microencapsulation was performed using the spray drying equipment (Gea-Niro®, model Mobile Minor 2000, Denmark) of stainless steel, with dispersion nozzle of 1 mm in diameter, where the microencapsulating synbiotic solution was atomized, with the support of a peristaltic pump (Watson Marlow®, model 520S, USA) at a dosing speed of 15 mL/min (10 to 27 mL/min), with an atomizing pressure of 1 to 2 bar, an inlet temperature of about 130°C to 160 °C and an outlet temperature of about 70°C to 80 °C.
The microencapsulated strains are referred in the present invention as SYN + strain name.

*Example 1 - Composition and process*

The microcapsule composition consisted of a probiotic (bacteria) solution of $10^{11}$ CFU/g, 5% inulin, 10% maltodextrin and 10 % gum Arabic. The composition was process according to the above methodology with an inlet temperature of about 160°C and outlet temperature of about 80°C, at 15ml/min and 2 bar.

3. Physical characterization of microcapsules

*a) Bacterial viability:*

A sample of the microencapsulated synbiotic was reconstituted in phosphate-buffered saline (PBS) solution pH 6.9, with vortex support to release the bacteria, serial dilutions (1:10) were made with phosphate-buffered saline (PBS) pH 6.9, the dilutions were seeded in duplicate, in plates with MRS agar, they were incubated at 37 °C for 48 h, and the count of colony forming units (CFU) was performed, the concentration was reported in CFU per gram (CFU/g), by the provisions of Official Mexican Standard NOM-092-SSA1-1994.

b) Encapsulation efficiency:

Represents the survival rate of bacteria after the drying process. It is calculated using the following equation (Arepally D. & Goswami T.K., 2019[5]):

$$\%EE = (N/N0) \times 100$$

Where N0 represents the Log10 CFU/g of viable bacteria present in the microencapsulating synbiotic solution before the drying process, and N represents the Log10 CFU/g of viable bacteria present in the microcapsules after the spray drying process.

c) Moisture content and water activity:

Was determined by placing a sample of the microencapsulated synbiotic on a moisture analyzer at 100 °C for 10 min, and wherein MC was calculated based on the difference in weight between before and after drying the capsules until they reach a constant weight. Water activity was determined with a water activity meter (Graigar, HBD5-MS2100Wa, CN) using a sample of the microencapsulate with temperature compensation and using the dew point method until equilibrium was reached. One gram of powdered product was used at a temperature of 21 $\pm$0.01 °C. [6]

d) Percentage of encapsulation yield:

Was calculated using the following equation:

$$\%EY = (M/M_0) \times 100$$

$M_0$ represents the grams of total solids present in the microencapsulating synbiotic solution, and M represents the grams of solids (microcapsules) recovered after the spray-drying process.

e) Tolerance to simulated gastrointestinal conditions in vitro:

The methodology followed the approach described by (Minekus et al, 2014) with slight modifications.
The survival of bacterial strains was assessed by sequentially exposing 1 g of synbiotic and 1 mL of bacterial suspension (separately for each strain) to simulated gastrointestinal fluids. For the oral phase, the sample was incubated 5 minutes in 9 mL of simulated salivary fluid (pH 7.0$\pm$0.2) at 37°C with agitation. For the gastric phase, 15 mL of simulated gastric fluid (pH 2.5$\pm$0.1) was added to the oral phase and incubated for 120 minutes under the same conditions as previous phase. For the intestinal phase, 80 mL of simulated intestinal fluid (pH 7.5$\pm$0.2) was added to the oral phase and incubated for 120 minutes.
At each simulated phase, a 1 mL aliquot was taken to quantify viable bacteria, as described in the Bacterial Viability section a).

4. Probiotic effect on human adenocarcinoma cells

a) Adhesion to HT-29 cells

The probiotic effect was studied on a in-vitro model of human adenocarcinoma cells of the HT-29-MTX lineage, as reported by Torres-Maravilla et al, 2016 [4]. Cells were seeded into 24-well cell culture plates ($5\times105$ cells/well). Plates were further incubated for an additional 21 days to allow for cellular differentiation. The microencapsulated bacterial strains were resuspended in DMEM medium ($2.5\times107$ CFU ) and added to each well.
After 2 hours incubation at 37°C, plates were washed with PBS and the number of adherent viable bacteria was determined by serial dilutions on MRS agar plates. Adhesion was expressed as the percentage of adherent bacteria relative to the initial number of bacteria, and the assay was performed in duplicate.

b) Example 2: Anti-proliferative activity:

The anti-proliferative activity of the probiotic strains was studied in HT-29, TC1, and Caco-2 lineages, based on the INRAe laboratory protocol, using the 3-(4,5-dimethylthiazol-2-yl)-2,5-diphenyltetrazolium bromide (MTS) assay, which relies on the reduction of MTS by actively growing cells.

Cells were cultured seeded into 96-well cell culture plates at a concentration of $2.4 \times 104$ cells/well and incubated for 24 hours at 37°C.

Bacterial strains were inoculated into each well at a multiplicity of infection (MOI) of 1:100, for microencapsulated bacterial strains, the synbiotic was placed at a MOI of 1:100 or 1:1000 5-Fluorouracil (5-FU) was used as a positive control, and PBS (20 μL) was used as a negative control. Plates were then incubated for 48 hours at 37 °C. After the incubation period, MTS was added to each well, and the plates were incubated for 3 hours at 37 °C. Absorbance was measured at 490 nm using the Tecan plate reader. The percentage of viable cells was calculated as follows: cell proliferation (%) = (OD of the experimental group/OD of the control group) $\times$ 100.

c) Example 3: Immunomodulatory properties of IL-8:

The immunomodulatory properties were evaluated by measuring the ability to suppress IL-8 secretion in TNF-$\alpha$-stimulated HT29 g+/- cells.

Cells were seeded into 96-well cell culture plates ($2.4 \times 106$ cells/well) and incubated for 24 hours at 37°C. Before bacterial inoculation, the medium in each well was removed and replaced with medium supplemented with TNF-$\alpha$.

Bacterial strains were inoculated into each well at a multiplicity of infection (MOI) of 1:40 Butyrate was used as a positive control, and DMEM medium without TNF-$\alpha$ was used as a negative control. Plates were then incubated for 48 hours at 37 °C.

IL-8 concentrations were determined using enzyme-linked immunosorbent assay (ELISA) analysis with the Human IL-8 ELISA MAX TM Kit Cat: 431501 from BioLegend, following the supplier's instructions.

## II. Results

### 2.1 Physical characterization

Table 1 below presents the physical characterization results of the microcapsules according to a preferred embodiment, namely example 1. The resulting microcapsules exhibit excellent viability after spray drying, with cell counts ranging from 9.50 to 10.36 Log10 CFU/g. All bacterial strains were encapsulated with high efficiency, with encapsulation rates ranging from 89.63% to 93.09%. The microcapsules also displayed relatively constant moisture content (4.12% to 5.4%) and water activity (0.23 to 0.32). The encapsulation yield ranged between 69.0% and 88.05%, indicating that some strains are more easily encapsulated than others.

**Table 1.** Physical characterization of synbiotic microcapsules obtained with an aqueous composition comprising according to example 1: maltodextrin 10% w/v, gum Arabic 10% w/v, inulin 5% w/v, total solids 25%, and processed using an inlet/outlet temperature of 160°C/80°C.

| Bacterial strain | | Viability after drying (Log10 CFU/g) | Encapsulation efficiency (%) | Moisture content (%) | Water activity | Encapsulation yield (%) |
|---|---|---|---|---|---|---|
| *Lactiplantibacillus plantarum* | SynLp115 | 10.01±0.53 | 89.63±3.00 | 5.02±0.34 | 0.27±0.02 | 84.97±1.85 |
| | SynLM20 | 10.29±0.17 | 90.76±1.58 | 4.12±0.20 | 0.23±0.01 | 88.05±1.91 |
| | SynLM19 | 9.50±0.64 | 93.09±3.45 | 5.0 ±0.60 | 0.312±0.13 | 69.0 ± 2.50 |
| *Lacticaseibacillus rhamnosus* | SynLM07 | 10.11±0.94 | 91.46±7.89 | 5.1 ±0.9 | 0.324±0.115 | 72.2 ± 4.9 |
| *Levilactobacillus brevis* | SynLBH1070 | 10.36±0.84 | 92.6±7.24 | 5.4 ±0.7 | 0.317 ±0.122 | 71.7± 4.1 |

The results presented here demonstrate that the microcapsule composition effectively protects various probiotic strains during air-drying, consistently achieving a minimum encapsulation efficiency of approximately 90%. This encapsulation efficiency reflects the survival rate of bacteria after the drying process. High moisture content has been linked to enhanced bacterial protection during spray drying, contributing to increased viability post-processing (see D. Arepally et al., 2020[1]). However, elevated moisture levels can negatively affect storage stability, which is why an optimal moisture content of 2-5% is normally preferred.

Water activity, another critical factor, is associated with microbiological and chemical degradation that can lead to instability during storage. For food powders, water activity levels of ≤0.6 are considered safe, minimizing the risk of spoilage and maintaining product quality [1]. In this invention, the microcapsule composition achieves a balance by combining a high moisture content, still within the optimal range, with low water activity. This unique combination offers microcapsules that not only ensure excellent microencapsulation efficiency-demonstrated by the high viability of bacteria after air drying-but also long-term stability during storage.

Specifically, it is believed that the relatively high moisture content aids in protecting bacteria during the air-drying process, thus preserving high bacterial viability. Simultaneously, the low water activity helps to prevent chemical and biological degradation of the probiotics during storage, enhancing their long-term viability.

Table 2 outlines various compositions tested according to this invention, along with their physical characterization. In these examples, different concentrations of maltodextrin and gum Arabic were evaluated using two *Lactiplantibacillus plantarum* strains (LM-20 and Lp-115), with total solid contents ranging from 25% to 30%. The effect of inlet temperature was also investigated, comparing temperatures of 130°C and 160°C, while inulin was kept at a constant concentration of 5% w/v.

**Table 2.** Physical characterization of different microcapsules compositions.

| Batch + strain | Process conditions: Inlet/outlet T° Pressure Dispersion speed | Composition (wall materials) Inulin 5% | Encapsulation efficiency (%) | Encapsulation yield (%) | Moisture content (%) | Water activity (aw) |
|---|---|---|---|---|---|---|
| A20 LM-20 | 130/80 ±2°C | Maltodextrin 10% Gum Arabic 10 % | 77.78±1.34 | 80.14±2.13 | 5.36±0.23 | 0.31±0.01 |
| B20 LM-20 | 2 bar 12-25 mL/min | Maltodextrin 15 % Gum Arabic 10 % | 65.71±1.76 | 75.23±1.45 | 5.12±0.13 | 0.28±0.02 |
| C20 LM-20 | | Maltodextrin 10% Gum Arabic 15 % | 45.71±1.24 | 60.32±1.52 | 4.89±0.34 | 0.32±0.01 |
| D20 LM-20 | 160/80 ±2°C | Maltodextrin 10% Gum Arabic 10 % | 90.82±1.72 | 88.23±1.54 | 4.25±0.15 | 0.21±0.02 |
| E20 LM-20 | 80°C 2 bar | Maltodextrin 15% Gum Arabic 10 % | 78.53±1.18 | 81.45±1.16 | 4.12±0.21 | 0.28±0.02 |
| F20 LM-20 | 12-25 mL/min | Maltodextrin 10% Gum Arabic 15 % | 56.01±1.26 | 71.32±1.12 | 4.22±0.15 | 0.28±0.01 |
| A115 Lp-115 | 130/80 ±2°C | Maltodextrin 10% Gum Arabic 10% | 81.48±1.12 | 79.87±1.19 | 4.12±0.19 | 0.24±0.02 |
| B115 Lp-115 | 80°C 2 bar | Maltodextrin 15 % Gum Arabic 10 % | 75.37±1.54 | 73.05±1.22 | 4.39±0.20 | 0.27±0.01 |
| C115 Lp-115 | 12-25 mL/min | Maltodextrin 10% Gum Arabic 15 % | 50.76±1.33 | 64.17±1.04 | 5.63±0.22 | 0.32±0.02 |
| D115 Lp-115 | 160/80 ±2°C | Maltodextrin 10% Gum Arabic 10 % | 89.19±2.40 | 85.01±1.68 | 5.03±0.23 | 0.25±0.02 |
| E115 Lp-115 | 80°C 2 bar | Maltodextrin 15% Gum Arabic 10 % | 81.97±2.05 | 80.05±1.45 | 4.76±0.17 | 0.26±0.01 |
| F115 Lp-115 | 12-25 mL/min | Maltodextrin 10% Gum Arabic 15 % | 66.51±1.14 | 65.33±1.11 | 5.12±0.14 | 0.28±0.02 |

The results show that all compositions, were able to provide microcapsules with the desired combination of relatively high moisture content (4.12-5.63%) and low water activity (0.21-0.32). However, the encapsulation efficiency and encapsulation yield are significantly influenced by the strain, the inlet temperature, and the total solids in the composition. For the tested conditions, the 160°C inlet temperature is preferred for all tested conditions and strains. Best results in these conditions are obtained with a total solids content of 25%. Preferably, the content of gum Arabic shall be inferior to 15% w/v to improve encapsulation yield and encapsulation efficiency.

The inventors also made further concluding tests wherein compositions with 20% solid content provided optimal process conditions and physical characteristics of synbiotic microcapsules of *Lactiplantibacillus plantarum* LM19, *Lacticaseibacillus rhamnosus* LM07, *Levilactobacillus brevis* LBH1070 (results not shown). In particular, the concentration of the material wall components was reduced, while keeping constant the prebiotic concentration.

Finally, the inventors verified the effect of the prebiotic concentration. Unexpectedly, increasing the concentration of inulin significantly altered the rheological properties of the solid suspension. It was observed that at a concentration of 10% w/v inulin, the viscosity of a 30% total solid suspension increased drastically. This rise in viscosity impeded the air-drying process, as it led to clogging at the equipment nozzles. Similarly, suspensions with a total solid concentration greater than 30% also became more difficult to handle, with clog formation occurring at approximately 35% solid content. This behavior highlights the importance of optimizing both inulin concentration and total solids content to ensure smooth processing without equipment issues.

### 2.2 Prebiotic concentration effect

The inventors further determined the effect of the prebiotic concentration, namely inulin on the microencapsulation process. The results in table 3 here after demonstrate that inulin is capable of regulating water activity of the microcapsules from the lower tested concentration (1%w/v), and to keep such water activity at a low desired value (0.206-0.253). However, in order to obtain optimal values of encapsulation efficiency and efficiency yield, as well as to ensure smooth processing, the concentration of inulin in the aqueous dispersion before drying shall be > 1 % w/v and <10% w/v, preferably the lower range concentration of inulin is ≥2% w/v, more preferably ≥3% w/v. The results also show that at higher concentrations inulin reduces the moisture content, and encapsulation yield. Hence, at a preferred embodiment, the upper range concentration of inulin is ≤8% w/v, more preferably ≤7 % w/v.

**Table 3.** Physical characterization of synbiotic microcapsules with different concentrations of prebiotic.

| Bacterial strain: *Lactiplantibacillus plantarum LM-20* | | | | | |
|---|---|---|---|---|---|
| Formulation: 200 mL (encapsulating suspension) Maltodextrin 10% Arabic gum 10% | Viability (before drying)/after drying) Log UFC/g | Encapsulation Efficiency (%) | Encapsulation Yield (%) | Moisture Content (%) | Water Activity (aw) |
| Inulin 1% | 10.38/7.94 | 76.56 | 61.40 | 5.95 | 0.253 |
| Inulin 3% | 10.44 / 8.69 | 83.20 | 76.93 | 5.08 | 0.206 |
| Inulin 5% | 10.53/9.72 | 92.26 | 89.86 | 4.72 | 0.206 |
| Inulin 7% | 10.47/9.50 | 90.69 | 71.33 | 4.80 | 0.208 |
| Drying Process Conditions | Inlet and Outlet Temperature and Pressure: 160±2 °C / 80±2 °C / 2 bar / Dispersion rate: 9-12 mL | | | | |

### 2.3 Storage stability at room temperature

Table 4 shows the initial viability after encapsulation and viability after 12 months and/or 24 months of storage under desiccator conditions at 25°C of synbiotic microcapsules according to example 1, notably comprising *Lactiplantibacillus plantarum, Lacticaseibacillus rhamnosus* and *Levilactobacillus brevis.*

**Table 4.** The bacterial stability of the microencapsulated synbiotic according to example 1 during storage.

| Bacterial strain | | Viability, day 0 (Log10 CFU/g) | Viability, 12 months (Log10 CFU/g) | Viability, 24 months (Log10 CFU/g) |
|---|---|---|---|---|
| *Lactiplantibacillus plantarum* | SynLp115 | 10.01±0.53 | 8.60±0.40 | 7.66±0.33 |
| | SynLM20 | 10.29±0.17 | 8.84±0.43 | 7.82±0.44 |
| | SynLM19 | 9.50±0.64 | 7.77±0.26 | ND |
| *Lacticaseibacillus rhamnosus* | SynLM07 | 10.11±0.94 | 7.07±0.45 | ND° |
| *Levilactobacillus brevis* | SynLBH1070 | 10.36±0.84 | 7.30±0.35 | ND |

The results demonstrate that the invention provides microcapsules with enhanced stability, capable of being stored at room temperature while maintaining high probiotic viability. Specifically, the viability of the encapsulated probiotics decreased by only 1 to 3 log orders after 12 months of storage at room temperature in a desiccator providing basic moisture protection with the presence of a desiccant (silica gel bag) at the bottom of the seal recipient. *Lactiplantibacillus plantarum* showed the best performance among all strains tested, with a viability reduction of no more than 2 log orders after 12 months and no more than 3 log orders after 24 months. In further studies (results not shown), the applicants also verified similar results on the viability of microcapsules that were stored for 12 months or more in other type of containers providing basic moisture protection, such as a tight sealed bag, or any close container adapted to the size of the product stored. The microcapsules of the invention will exhibit therefore such enhanced viability when appropriately stored according to normal food product and medicament packaging practices. Off course, including silica gel bags will be recommended if the microcapsules are store in bulk in a container.

These findings highlight the ability of the microcapsules to preserve high bacterial viability over extended storage periods, without the need for stabilizers, special coatings, or complex formulations. Importantly, for all strains tested, the microcapsules retained more than 6 log10 CFU/g after 12 months of storage, which is the minimum required concentration for a probiotic product to deliver a beneficial effect upon ingestion. This underlines the practical application of the invention for producing stable probiotic products suitable for long-term storage at room temperature.

### 2.4 Tolerance to simulated gastrointestinal conditions in vitro

Table 5 presents the bacterial viability of various strains exposed to simulated human digestive tract conditions. All microencapsulated strains (e.g., SynLp115, Syn LM20) demonstrated significantly higher tolerance to gastric conditions compared to their non-encapsulated counterparts (data not shown). These results confirm that the microcapsule composition of the invention enhances the survival of multiple probiotic strains through human digestion.

However, survival rates varied across strains, suggesting that some strains are inherently more resilient. The differences in final viability appear to partially correlate with the encapsulation yield for each strain, indicating that this parameter might help determinate how well a particular strain survives digestion when microencapsulated according to this invention. Indeed, the strain with the best encapsulation yield was LM20, followed by Lp115, LM07, LBH1070, and LM19. The best viability results in simulated gastric conditions are also provided by LM20, followed by Lp115 and LM07.

**Table 5.** Bacterial viability of the synbiotic after 4 h *in vitro* digestion test.

| Bacterial strain | | Initial viability (Log10 CFU/g) | Final viability (Log10 CFU/g) | Final viability (%) |
|---|---|---|---|---|
| *Lactiplantibacillus plantarum* | SynLp115 | 10.55±0.46 | 9.67±0.55 | 91.93 |
| | SynLM20 | 10.78±0.42 | 9.97±0.60 | 92.66 |
| | SynLM19 | 10.21 ± 0.04 | 7.15 ± 0.05 | 70.0 |
| *Lacticaseibacillus rhamnosus* | SynLM07 | 9.76 ± 0.37 | 7.30 ± 0.29 | 74.9 |
| *Levilactobacillus brevis* | SynLBH1070 | 10.05 ± 0.36 | 6.76 ± 0.02 | 67.3 |

### 2.5 Validation of probiotic effect on human adenocarcinoma cell lines

The symbiotic microcapsules developed in this invention were tested on human adenocarcinoma cell lines to assess their potential probiotic effects on cancers originating from glandular epithelial tissues. Specifically, this model allows for the evaluation of the probiotic effects on colorectal cancer, the most common form of this type of cancer. The tests focused on observing the ability of the microencapsulated probiotics to adhere to HT-29 cells, a widely used model for colorectal cancer, as well as their influence on tumor cell proliferation and modulation of IL-8 expression, an inflammatory cytokine involved in cancer progression.

The results, presented in Table 6 below, highlight the potential of the symbiotic microcapsules to adhere to HT-29 cells and modulate key cancer-related processes.

Table 6. Application of the synbiotic on human adenocarcinoma cell lines.

| Bacterial strain | | Adhesion capacity to HT-29 cells (%) | Proliferation of tumor cells | | | Capacity to modulate IL-8 in HT-29 cells (%) |
|---|---|---|---|---|---|---|
| | | | HT-29 (%) | TC1 (%) | Caco-2 (%) | |
| *Lactiplantibacillus plantarum* | SynLp115 | 21.19 | 47.98 | 53.89 | 79.09 | 38.12 |
| | SynLM20 | 22.51 | 40.66 | 47.98 | 59.48 | 33.75 |
| | SynLM19 | 6.60±1.0 | 88.30±3.75 | 54.46±12.25 | 37.77±5.25 | 12.86±1.08 |
| *Lacticaseibacillus rhamnosus* | SynLM07 | 6.73±2.0 | 21.11±0.87 | 15.98±0.58 | 33.52±1.45 | 16.17±3.20 |
| *Levilactobacillus brevis* | SynLBH1070 | 12.73±3.51 | 76.36±8.44 | 23.05±0.70 | 35.26±1.42 | 9.53±2.99 |

Specifically, all tested microencapsulated probiotics showed adhesion capacity, reflecting their ability to colonize the HT-29 cells and their potential to be used as a treatment for targeting tumors. In general, the effect varied significantly between the tested strains, suggesting that some strains are more adept at modulating tumor cell proliferation and IL-8 expression. These results indicate complex interaction mechanisms that depend on the specific strain and the cell lines tested. Notably, *Lactiplantibacillus plantarum* strains SynLp115 and Syn LM19 showed promising results, particularly in their ability to adhere to HT-29 cells and modulate IL-8 levels.

**List of references**

[1] Arepally et al, Studies on survivability, storage stability of encapsulated spray dried probiotic powder, Current Research in Food Science, Volume 3, 2020, Pages 235-242, https://doi.org/10.1016/j.crfs.2020.09.001.

[2] Sandra V. Avila-Reyes et al, Protection of L. rhamnosus by spray-drying using two prebiotics colloids to enhance the viability, Carbohydrate Polymers, Volume 102, 2014, Pages 423-430, https://doi.org/10.1016/j.carb pol.2013.11.033.

[3] Hernández-Delgado, N.C. et al. Antioxidant and Anti-Inflammatory Properties of Probiotic Candidate Strains Isolated during Fermentation of Agave (Agave angustifolia Haw). Microorganisms 2021, 9, 1063. https://doi.org/10.3390/microorganisms9051063

[4] Torres-Maravilla, Edgar et al. Evaluation of pulque sediment (xaxtle) as a starter culture in order to obtain a low glycemic index baked product. Agrociencia [online]. 2016, vol.50, n.2 [citado 2024-09-16], pp.183-200.

[5] Arepally, D., & Goswami, T. K. (2019). Effect of inlet air temperature and gum Arabic concentration on encapsulation of probiotics by spray drying. LWT, 99, 583-593. https://doi.org/10.1016/j.lwt.2018.10.022

[6] Márquez-Lemus M, Cano-Sampedro E, Alamilla-Beltrán L, & Mora-Escobedo R. (2023). Efecto del mucilago del pericarpio de tuna (Opuntia robusta Wendl. var.robusta) en el secado por aspersión de jugo de tuna. Revista Bio Ciencias. https://doi.org/10.15741/revbio.10.e1476

**Claims**

1. Synbiotic microcapsules, wherein such microcapsules are obtained by spray-drying and comprise a composition consisting essentially of a probiotic and a mixture of:

   - a prebiotic selected from the group consisting of fructooligosaccharides (FOS), galactooligosaccharides (GOS), xylooligosaccharides (XOS), and arabinoxylans and mixtures thereof;
   - maltodextrin, and
   - a protective colloid selected from the group consisting of gum Arabic, starch, modified starches, pectin, xanthan gum and mixtures thereof.

2. Synbiotic microcapsules according to claim 1, wherein the prebiotic is inulin.

3. Synbiotic microcapsules according to one of the preceding claims, presenting a moisture content of 4% to 6%w/w, and a water activity ≤ 0.4, preferably ≤0.35.

4. Synbiotic microcapsules according to one of the preceding claims, wherein the prebiotic is inulin and the protective colloid is gum Arabic or is mainly composed of gum Arabic.

5. Synbiotic microcapsules according to one of the preceding claims, wherein the microcapsules present enhanced storage stability at room temperature such that an initial viability counting of such symbiotic microcapsules decreases less than 4 log CFU/g after 12 months storage at 25°C, at preferably no more than 3 log CFU/g.

6. Synbiotic microcapsules according to one of the preceding claims, wherein the composition presents the following content in w/v % when present in aqueous solution:

   1 - 10% of prebiotic, more preferably 2-8%, more preferably 3-7 %;
   5 - 15% of maltodextrin, preferably 5-10%
   5 - 15% of protective colloid, preferably 5-10%; and

   wherein the total solid content in such aqueous solution, excluding the probiotic, is 20 - 30 %w/v, preferably 20-25% w/v.

7. Synbiotic microcapsules according to one of the preceding claims, wherein the composition presents the following content in w/v % when present in aqueous solution:

   3 - 7 % of inulin as prebiotic, preferably 4-6%;
   5 - 15% of maltodextrin, preferably 5-10%;
   5 - 10% of gum Arabic as protective colloid, and

   wherein the total solid content in such aqueous solution, excluding the probiotic, is 20-25% w/v.

8. Synbiotic microcapsules according to one of the preceding claims, wherein such probiotic is a lactic acid bacteria, a bifidobacteria or a mixture thereof, and wherein such probiotic is preferably *Lacticaseibacillus rhamnosus, Levilactobacillusbrevis*, *Lactiplantibacillus plantarum* or a mixture thereof.

9. Synbiotic microcapsules according to one of claims 1 to 8 for use in the treatment and/or prevention of chronic inflammatory bowel diseases, such as ulcerative colitis.

10. Synbiotic microcapsules according to one of claims 1 to 8 for use in the treatment and/or prevention of gastrointestinal diseases and disorders including colorectal cancer, and/or for their use as probiotics, antioxidants, antimicrobials and/or antiinflammatories.

11. Synbiotic microcapsules for use according to one of claims 9 to 10, comprising *Lactiplantibacillus plantarum* as probiotic.

12. Food product, nutraceutical or medicament with a composition including synbiotic microcapsules according to one of claims 1 to 8.

13. Process for obtaining synbiotic microcapsules according to one of claims 1 to 8, comprising the following actions:

   a) Preparing an aqueous dispersion including all the microcapsule composition components, and
   b) Air spray-drying such aqueous suspension.

14. Process according to claim 13, wherein in b) air spray-drying is performed with an inlet temperature of 130°C to 170°C, preferably 145°C-165°C, and an outlet temperature of 65°C to 85°C, preferably 70°C-80°C.

15. Process according to one of claims 13 or 14, wherein in b) air spray-drying is performed using a dosing speed of 8 to 27 mL/min, and/or an atomizing pressure of 1 to 2 bar, and/or using natural air as heating fluid.

**Fig. 1**

**Fig. 2**

Europäisches
Patentamt

European
Patent Office

Office européen
des brevets

# EUROPEAN SEARCH REPORT

**Application Number**

EP 24 30 6864

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | NEUENFELDT NAIARA HENNIG ET AL: "Effects of blueberry extract co-microencapsulation on the survival of Lactobacillus rhamnosus", LWT- FOOD SCIENCE AND TECHNOLOGY, vol. 155, 1 February 2022 (2022-02-01), page 112886, XP093266148, United Kingdom ISSN: 0023-6438, DOI: 10.1016/j.lwt.2021.112886 | 1-10, 12-15 | INV. A61K9/16 A23L33/135 |
| Y | * paragraph 2.4; page 2 * * table 1 * | 1-15 | |
| Y | AVILA-REYES SANDRA V ET AL: "Protection ofL.rhamnosusby spray-drying using two prebiotics colloids to enhance the viability", CARBOHYDRATE POLYMERS, APPLIED SCIENCE PUBLISHERS , LTD BARKING, GB, vol. 102, 4 December 2013 (2013-12-04), pages 423-430, XP028607305, ISSN: 0144-8617, DOI: 10.1016/J.CARBPOL.2013.11.033 * Item 2.1 and 2.2.; page 424 * | 1-15 | |
| Y | CHEN CHEN ET AL: "Advances in oligosaccharides and polysaccharides with different structures as wall materials for probiotics delivery: A review", INTERNATIONAL JOURNAL OF BIOLOGICAL MACROMOLECULES, ELSEVIER BV, NL, vol. 277, 31 August 2024 (2024-08-31), XP087595880, ISSN: 0141-8130, DOI: 10.1016/J.IJBIOMAC.2024.134468 [retrieved on 2024-08-31] * the whole document * | 1-15 | |

**TECHNICAL FIELDS SEARCHED (IPC)**

A61K
A23L

The present search report has been drawn up for all claims

| Place of search | Date of completion of the search | Examiner |
|---|---|---|
| Munich | 16 April 2025 | Schüle, Stefanie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

....................................................................

& : member of the same patent family, corresponding document

EPO FORM 1503 03.82 (P4C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019144979 A
- WO 2015063090 A

**Non-patent literature cited in the description**

- **AREPALLY et al.** Studies on survivability, storage stability of encapsulated spray dried probiotic powder. *Current Research in Food Science*, 2020, vol. 3, 235-242, https://doi.org/10.1016/j.crfs.2020.09.001
- **SANDRA V. AVILA-REYES et al.** Protection of L. rhamnosus by spray-drying using two prebiotics colloids to enhance the viability. *Carbohydrate Polymers*, 2014, vol. 102, 423-430, https://doi.org/10.1016/j.carbpol.2013.11.033
- **HERNÁNDEZ-DELGADO, N.C. et al.** Antioxidant and Anti-Inflammatory Properties of Probiotic Candidate Strains Isolated during Fermentation of Agave (Agave angustifolia Haw). *Microorganisms*, 2021, vol. 9, 1063, https://doi.org/10.3390/microorganisms9051063
- **TORRES-MARAVILLA, EDGAR et al.** Evaluation of pulque sediment (xaxtle) as a starter culture in order to obtain a low glycemic index baked product. *Agrociencia*, 2016, vol. 50 (2), 183-200
- **AREPALLY, D.** ; **GOSWAMI, T. K.** Effect of inlet air temperature and gum Arabic concentration on encapsulation of probiotics by spray drying. *LWT*, 2019, vol. 99, 583-593, https://doi.org/10.1016/j.lwt.2018.10.022
- **MÁRQUEZ-LEMUS M** ; **CANO-SAMPEDRO E** ; **ALAMILLA-BELTRÁN L** ; **MORA-ESCOBEDO R.** Efecto del mucilago del pericarpio de tuna (Opuntia robusta Wendl. var.robusta) en el secado por aspersión de jugo de tuna. *Revista Bio Ciencias.*, 2023, https://doi.org/10.15741/revbio.10.e1476